# EUROPÄISCHE PATENTSCHRIFT

(11) **EP 4 013 806 B1**
(45) Veröffentlichungstag und Bekanntmachung des Hinweises auf die Patenterteilung: **26.06.2024**
(21) Anmeldenummer: 20753359.7
(22) Anmeldetag: 10.08.2020
(51) Int. Cl.: C08G 71/04, C12N 9/78, C12P 13/02, C12P 13/00, C12P 7/04

(54) **VERFAHREN ZUM ABBAU VON POLYETHER-POLYURETHAN**
METHOD FOR DEGRADING POLYETHER POLYURETHANE
PROCÉDÉ DE DÉCOMPOSITION DE POLYÉTHER-POLYURÉTHANE

(30) Priorität: 16.08.2019 EP 19192115
(43) Veröffentlichungstag der Anmeldung: 22.06.2022
(73) Patentinhaber: Covestro Deutschland AG, 51373 Leverkusen (DE)
(72) Erfinder: REISKY, Lukas, 50996 Köln (DE); JÄGER, Gernot, 50733 Köln (DE)
(74) Vertreter: Levpat
(86) Internationale Anmeldenummer: PCT/EP2020/072333
(87) Internationale Veröffentlichungsnummer: WO 2021/032513

(56) Entgegenhaltungen:
- EP-A2- 2 825 586
- WO-A1-2006/019095
- HYNEK BENES ET AL: "Utilization of Natural Oils for Decomposition of Polyurethanes", JOURNAL OF POLYMERS AND THE ENVIRONMENT ; FORMERLY: 'JOURNAL OF ENVIRONMENTAL POLYMER DEGRADATION', KLUWER ACADEMIC PUBLISHERS-PLENUM PUBLISHERS, NE, Bd. 20, Nr. 1, 30. Juli 2011 (2011-07-30), Seiten 175-185, XP035017444, ISSN: 1572-8900, DOI: 10.1007/S10924-011-0339-8 in der Anmeldung erwähnt

## Beschreibung

Die vorliegende Erfindung betrifft ein Verfahren, mit dem Polyurethane, die aus Polyetherpolyolen und Isocyanaten aufgebaut wurden, zu Polyetherpolyolen und Aminen abgebaut werden können.

Polyurethane werden in großen Mengen unter anderem zur Herstellung von harten und weichen Schäumen verwendet. Wenn diese Materialien am Ende ihrer Lebensdauer verbrannt werden, wird CO₂ freigesetzt. Da die Rohstoffe zur Herstellung der Polyurethane überwiegend oder vollständig aus Erdöl hergestellt werden, führt die Verbrennung von Polyurethanen zu einem Anstieg des CO₂-Gehaltes der Atmosphäre. Deswegen ist eine möglichst umfassende stoffliche Wiederverwertung von Polyurethanabfällen sehr wünschenswert.

Polyurethane werden aus einer Isocyanatkomponente und einer Polyolkomponente aufgebaut. Diese bilden durch eine Additionsreaktion die Urethangruppe, welche die beiden Komponenten verbindet und so das Polymernetzwerk aufbaut. Für die Herstellung von Schäumen werden vorwiegend Isocyanate mit aromatisch gebundenen Isocyanatgruppen, insbesondere Toluylendiisocyanat, Methylendiphenylisocyanat und mehrkernige Derivate des Methylendiphenylisocyanats eingesetzt. Als Polyolkomponente werden häufig Polyetherpolyole verwendet.

Zur Rückgewinnung der Polyetherpolyole aus Polyurethanen existieren bereits Verfahren, die zum Teil auch schon großtechnisch erprobt werden. Diese Verfahren basieren auf der Umsetzung des Polyurethans mit niedermolekularen Alkoholen wie z.B. Glykol. Hierbei findet ein Austausch des Polyetherpolyols gegen den niedermolekularen Alkohol statt. Dieser Austausch eines Polyols, das Bestandteil einer Urethangruppe ist, gegen ein anderes Polyol wird in der vorliegenden Patentanmeldung analog zu einer Umesterung als "Umurethanisierung" bezeichnet. Reaktionsprodukte einer solchen Umurethanisierung sind der zur Herstellung des Polyurethans verwendete Polyether und ein Urethan, das aus dem zur Synthese des Polyurethans verwendeten aromatischen Polyisocyanat und dem zur Umurethanisierung verwendeten niedermolekularen Alkohol abgeleitet ist. Das neu gebildete Urethan ist niedermolekular, da zur Umurethanisierung gegenüber dem Polyethergehalt des Polyurethans ein hoher molarer Überschuss des niedermolekularen Polyols eingesetzt wird, so dass ein Großteil der niedermolekularen Polyolmoleküle nur mit einem Molekül, das von dem zur Herstellung des Polyetherpolyurethans verwendeten Isocyanat abgeleitet ist, reagiert. Details und Varianten dieses Verfahrens werden in Simon et al. (2018), Waste Management, 76: 147-171 beschrieben.

Stofflich verwertet wird allerdings nur das aus dem Polyurethan freigesetzte Polyetherpolyol. Das im Zuge der Umurethanisierung gebildete niedermolekulare Urethan ist ein Nebenprodukt, für das es bisher keine zufriedenstellende Verwendung gibt. Simon et al. (2014), Journal of Material Cycles and Waste Management, 16: 525-523 beschreiben ein Verfahren, bei dem der nicht abreagierte niedermolekulare Alkohol destillativ von dem Umurethanisierungsprodukt abgetrennt wurde. Der Destillationsrückstand bestand aus einem schlecht definierten Gemisch aromatischer Amine und niedermolekularer Urethane. Dieser Rückstand konnte als Initiator für die Synthese von Polyetherpolyolen eingesetzt werden. Für den Einsatz des Destillationsrückstandes in weiteren Anwendungsfeldern ist die Vielfalt der darin vorliegenden Verbindungen nachteilig.

Spiegelbildlich zu dem oben beschriebenen Verfahren wird in Beneš, H., Černá, R., Ďuračková, A., & Lätalovä, P. (2012). Utilization of natural oils for decomposition of polyurethanes. Journal of Polymers and the Environment, 20(1), 175-185 ein Verfahren beschrieben, bei dem Fischöl bzw. Rizinusöl als Alkohole für die Umurethanisierung eingesetzt werden. Dieses Verfahren zielt auf die Gewinnung des niedermolekularen Urethans als Ausgangsstoff für weitere chemische Reaktionen ab. Da die verwendeten Alkohole stark hydrophob sind, liegt dieses Produkt am Ende des Verfahrens in einer Phase zusammen mit dem freigesetzten Polyether vor.

Der vorliegenden Erfindung lag die Aufgabe zugrunde, ein Verfahren bereitzustellen, das die möglichst vollständige Zerlegung von Polyetherpolyurethanen in chemisch möglichst genau definierte Verbindungen ermöglicht.

Gelöst wird diese Aufgabe durch ein Verfahren enthaltend die Schritte
a) Umurethanisierung eines Polyetherpolyurethans mit wenigstens einem niedermolekularen Alkohol, wobei Polyetherpolyole und niedermolekulare Urethane entstehen; und
b) die enzymatische Spaltung der in Verfahrensschritt a) entstandenen niedermolekularen Urethane, wobei wenigstens ein Amin und der wenigstens eine, in Verfahrensschritt a) eingesetzte niedermolekulare Alkohol freigesetzt werden.

Der Verfahrensschritt a) hat zwei Ziele: (i) Das zur Synthese des Polyurethans verwendete Polyetherpolyol soll als isolierbare Verbindung aus dem Polyurethan freigesetzt werden. (ii) Das zur Synthese des Polyurethans verwendete Isocyanat soll als Bestandteil eines niedermolekularen Urethans vorliegen. Das besagte niedermolekulare Urethan ist - im Gegensatz zum Polyurethan mit seinem hohen Molekulargewicht - durch sein geringeres Molekulargewichts und die dadurch bessere Löslichkeit als Substrat für die in Verfahrensschritt b) erfolgende enzymatische Spaltung gut geeignet.

In Verfahrensschritt b) werden durch enzymatische Spaltung der niedermolekularen Urethane ein Amin und der in Verfahrensschritt a) zur Umurethanisierung eingesetzte niedermolekulare Alkohol freigesetzt. Zusätzlich wird in diesem Verfahrensschritt CO₂ freigesetzt. Diese Verbindungen können durch geeignete Trennungsverfahren isoliert und anschließend weiter verwendet werden. Hierbei ist es bevorzugt, den freigesetzten niedermolekularen Alkohol erneut für die in Verfahrensschritt a) erfolgende Umurethanisierung einzusetzen. Das freigesetzte Amin steht als reiner und gut definierter Ausgangsstoff für neue Synthesen zur Verfügung.

### Polyetherurethan

Ein Polyurethan ist eine Verbindung, die aus Polyolen und Polyisocyanaten aufgebaut ist. Die Gesamtheit aller zum Aufbau des Polyurethans eingesetzten Polyole wird in dieser Anmeldung auch als "Polyolkomponente" bezeichnet. Die Gesamtheit aller zum Aufbau des Polyurethans verwendeten Polyisocyanate wird in der vorliegenden Anmeldung als "Isocyanatkomponente" bezeichnet. Jeweils eine Hydroxylgruppe des Polyols und bildet mit je einer Isocyanatgruppe des Polyisocyanats durch eine Additionsreaktion eine Urethangruppe und vernetzt so die Aufbaukomponenten des Polyurethans.

Das nach dem erfindungsgemäßen Verfahren abzubauende Polyurethan ist ein Polyetherpolyurethan. Dieser Begriff bezeichnet Polyurethane, deren Polyolkomponente Polyetherpolyole enthält. Bevorzugt sind wenigstens 40 Gew.-% der in der Polyolkomponente enthaltenen Hydroxylgruppen Bestandteile von Polyetherpolyolen. Stärker bevorzugt sind dies wenigstens 60 Gew.-%, noch stärker bevorzugt wenigstens 80 Gew.-% und am stärksten bevorzugt wenigstens 95 gew.-%. Es ist erfindungsgemäß möglich, dass ein Polyetherpolyurethan unter Einhaltung der vorgenannten Mengenanteile der Polyetherpolyole auch noch weitere Polyole als Aufbaukomponenten enthält. Dies sind vorzugsweise Polyesterpolyole.

Grundsätzlich können Isocyanatgruppen auch mit anderen funktionellen Gruppen reagieren, die Zerewitinoff-aktive Wasserstoffatome enthalten. Solche funktionellen Gruppen sind insbesondere Aminogruppen und Thiolgruppen. In diesem Fall entstehen durch die Additionsreaktion Harnstoffgruppen bzw. Thiourethangruppen. Bei einem "Polyetherurethan" gemäß der vorliegenden Anmeldung liegt aber der Anteil von Urethan- und Harnstoffbindungen an der Gesamtmenge Urethan-, Harnstoff- und Thiourethanbindungen bei wenigstens 60 Mol-%, bevorzugt wenigstens 80 Mol-% und stärker bevorzugt wenigstens 90 Mol-%. Der Anteil der Urethanbindungen an der Gesamtmenge der Urethan-, Harnstoff- und Thiourethanbindungen liegt wenigstens bei 20 Mol-%, bevorzugt bei wenigstens 40 Mol-% und stärker bevorzugt bei wenigstens 60 Mol-%.

Weiterhin weist ein "Polyurethan" im Sinne der vorliegenden Anmeldung pro Molekül wenigstens 3, bevorzugt wenigstens 5 Urethangruppen auf. Die hierdurch bewirkte Vernetzung mehrerer Moleküle der beteiligten Aufbaukomponenten führt zu einem hohen Molekulargewicht des Polyurethans. Deswegen liegt das zahlenmittlere Molekulargewicht eines nach dem erfindungsgemäßen Verfahren abzubauenden Polyurethans vorzugsweise bei wenigstens 1350 g/Mol.

### Polyetherpolyol

Der Begriff "Polyetherpolyol" ist dem Fachmann gut bekannt. Hierbei handelt es sich um Polyether mit einer durchschnittlichen Hydroxylfunktionalität zwischen 1,5 und 6,0. Bevorzugt ist das im Polyetherpolyurethan enthaltene Polyetherpolyol ein Polyadditionsprodukt eines oder mehrerer Alkylenoxide mit 2 bis 4 Kohlenstoffatomen unter Verwendung mindestens eines Startermoleküls, das 2 bis 8, vorzugsweise 2 bis 6, reaktive Wasserstoffatome gebunden enthält.

Bevorzugte Alkylenoxide sind Styroloxid, Ethylenoxid, Propylenoxid, Tetrahydrofuran, Butylenoxid, Epichlorhydrin. Stärker bevorzugt sind 1,3-Propylenoxid, 1,2- bzw. 2,3-Butylenoxid und Styroloxid. Besonders bevorzugt sind Ethylenoxid und 1,2-Propylenoxid. Die Alkylenoxide können einzeln, alternierend nacheinander oder als Mischungen verwendet werden.

Als Startermoleküle der Polyaddition werden bevorzugt Wasser, organische Dicarbonsäuren, aliphatische und aromatische, gegebenenfalls N-mono-, N,N- und N,N'-dialkylsubstituierte, Diamine mit 1 bis 4 Kohlenstoffatomen im Alkylrest, zweiwertige Alkohole und mehrwertige Alkohole

Bevorzugte organische Dicarbonsäuren sind Bernsteinsäure, Adipinsäure, Phthalsäure und Terephthalsäure.

Bevorzugte Diamine sind mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 2,2'-, 2,4'- und 4,4'-Diaminodiphenylmethan.

Bevorzugte zwei und mehrwertige Alkohole sind Ethandiol, 1,2- und 1,3-Propandiol, Diethylenglykol, Dipropylenglykol, 1,4-Butandiol, 1,6-Hexandiol, Triethanolamin, Bisphenole, Glycerin, Trimethylolpropan, Pentaerythrit, Sorbit und Saccharose.

### Isocyanat

Für den Abbau durch das erfindungsgemäße Verfahren sind grundsätzlich Polyurethane geeignet, deren Isocyanatkomponente Isocyanate mit aliphatisch, cycloaliphatisch, aromatisch oder araliphatisch gebundenen Isocyanatgruppen enthält.

Bei einem Isocyanat mit aliphatisch gebundenen Isocyanatgruppen sind alle Isocyanatgruppen an ein Kohlenstoffatom gebunden, das Teil einer offenen Kohlenstoffkette ist. Diese kann an einer oder mehreren Stellen ungesättigt sein. Die aliphatisch gebundene Isocyanatgruppe oder - im Fall von Polyisocyanaten - die aliphatisch gebundenen Isocyanatgruppen sind vorzugsweise an den terminalen Kohlenstoffatomen der Kohlenstoffkette gebunden.

Erfindungsgemäß besonders geeignete Polyisocyanate mit aliphatisch gebundenen Isocyanatgruppen sind 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan und 1,10-Diisocyanatodecan.

Bei einem Isocyanat mit cycloaliphatisch gebundenen Isocyanatgruppen sind alle Isocyanatgruppen an Kohlenstoffatome gebunden, die Teil eines geschlossenen Rings aus Kohlenstoffatomen sind. Dieser Ring kann an einer oder mehreren Stellen ungesättigt sein, solange er durch das Vorliegen von Doppelbindungen keinen aromatischen Charakter erhält.

Erfindungsgemäß besonders geeignete Polyisocyanate mit cycloaliphatisch gebundenen Isocyanatgruppen sind 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan Isophorondiisocyanat; (IPDI), 1-Isocyanato-1-methyl-4(3)-isocyanatomethylcyclohexan, 2,4'- und 4,4'-Diisocyanatodicyclohexylmethan(H12MDI), 1,3-und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyl-dicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan und 1,3-Dimethyl-5,7-diisocyanatoadamantan .

Bei einem Isocyanat mit araliphatisch gebundenen Isocyanatgruppen sind alle Isocyanatgruppen an Methylenreste gebunden, der ihrerseits an einen aromatischen Ring gebunden sind.

Erfindungsgemäß besonders geeignete Polyisocyanate mit araliphatisch gebundenen Isocyanatgruppen sind 1,3- und 1,4-Bis-(iso¬cyanatomethyl)benzol (Xylylendiisocyanat); XDI), 1,3- und 1,4-Bis(1-isocyanato-1-methyl¬ethyl)-benzol (TMXDI) und Bis(4-(1-isocyanato-1-methylethyl)phenyl)-carbonat.

Erfindungsgemäß kann die polymerisierbare Zusammensetzung beliebige Mischungen der oben genannten Isocyanate in monomerer und/oder oligomerer Form enthalten.

Bei einem Isocyanat mit aromatisch gebundener Isocyanatgruppe sind alle Isocyanatgruppen direkt an Kohlenstoffatome gebunden, die Teil eines aromatischen Ringes sind.

Erfindungsgemäß besonders geeignete Isocyanate mit aromatisch gebundenen Isocyanatgruppen sind Toluylendiisocyant (TDI), Methylendiphenylisocyanat (MDI) und Naphthylendiisocyanat.

Der Begriff "Toluylendiisocyanat" bezeichnet Toluylen-2,4-diisocyanat (2,4-TDI), Toluylen-2,6-diisocyanat (2,6-TDI) sowie beliebige Gemische der beiden Isomere. Der Begriff "Methylendiphenylisocyanat" bezeichnet alle Isomeren des MDI, insbesondere 2,2'-Diphenylmethandiisocyanat, 2,4'-Diphenylmethandiisocyanat, 4,4'-Diphenylmethandiisocyanat , alle Gemische, die wenigstens zwei der vorgenannten Isomere enthalten, sowie mehrkernige Derivate des MDI.

Der Begriff "Naphthylendiisocyanat" bezeichnet, 1,4-Naphthylendiamin, 1,5-Naphthylendiamin und 1,6-Naphthylendiamin sowie beliebige Gemische der vorgenannten Isomere.

Bevorzugt werden aber Polyetherpolyurethane abgebaut, deren Isocyanatkomponente Isocyanate mit aromatisch gebundenen Isocyanatgruppen enthält oder die aus solchen Isocyanaten besteht. Besonders bevorzugt enthält die Isocyanatkomponente des Polyetherpolyurethans TDI, MDI oder beliebige Gemische der beiden oben genannten Isocyanate.

In einer besonders bevorzugten Ausführungsform der vorliegenden Erfindung sind wenigstens 50 Mol-% der in der Isocyanatkomponente enthaltenen Isocyanatgruppen Bestandteil von TDI und/oder MDI. Stärker bevorzugt sind dies wenigstens 65 Mol-% und noch stärker bevorzugt wenigstens 80 Mol-%.

### Niedermolekularer Alkohol

Damit das niedermolekulare Urethan aus Verfahrensschritt a) als Substrat für eine enzymatische Spaltung der Urethanbindung geeignet ist, darf der niedermolekulare Alkohol kein zu hohes Molekulargewicht haben. Dieses liegt bei höchstens 500 g/Mol und am stärksten bevorzugt bei höchstens 200 g/Mol.

Weiterhin ist es vorteilhaft, niedermolekulare Alkohole zu verwenden, deren Urethane mit dem aus dem Polyetherurethan freigesetzten Polyetherpolyol getrennte Phasen bilden. Hierbei können auch solche niedermolekularen Alkohole eingesetzt werden, deren Ester nach Zugabe eines weiteren Lösungsmittels getrennte Phasen bilden. Hierdurch wird die Ausbildung einer separaten Polyetherphase begünstigt, so dass das freigesetzte Polyetherpolyol einfach aus dem Reaktionsgemisch abgetrennt werden kann. Aus diesem Grund sind niedermolekulare Alkohole mit einer höheren Polarität bevorzugt.

Der niedermolekulare Alkohol enthält wenigstens 2 Hydroxylgruppen pro Molekül und hat ein Molekulargewicht von höchstens 500 g/Mol, stärker bevorzugt höchstens 200 g/Mol.

Aus technischer Sicht sind weiterhin niedermolekulare Alkohole besonders geeignet, die einen niedrigen Schmelzpunkt haben, so dass keine Gefahr besteht, dass sie in den Rohrleitungen der Anlage erstarren und diese so verstopfen. Deswegen werden bevorzugt niedermolekulare Alkohole mit einem Schmelzpunkt von höchstens 45 °C verwendet. Stärker bevorzugt weisen die verwendeten niedermolekularen Alkohole einen Schmelzpunkt von höchstens 20 °C auf.

Der wenigstens eine niedermolekulare Alkohol ist vorzugsweise ausgewählt aus der Gruppe bestehend aus Methanol, Ethanol, Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol, Polyethylenglykol 400 und Mischungen von zwei oder mehr der vorgenannten Alkoholen. Stärker bevorzugt ist er ausgewählt aus der Gruppe bestehend aus Ethylenglykol, Diethylenglykol, Propylenglykol, Dipropylenglykol, Methylglykol, Triethylenglykol und Mischungen von zwei oder mehr der vorgenannten Alkohole besonders bevorzugt. Ganz besonders bevorzugt ist der niedermolekulare Alkohol Diethylenglykol.

### Reaktionsprodukte

Im ersten Verfahrensschritt entsteht ein freier Polyether, der durch physikalische Verfahren aus dem Reaktionsgemisch abgetrennt werden kann.

Bei Verwendung eines niedermolekularen Alkohols von hinreichend hoher Polarität bildet sich ein Gemisch aus zwei Phasen. Die weniger polare Phase besteht weitgehend aus dem Polyether. Die andere, polarere Phase enthält den nicht verbrauchten niedermolekularen Alkohol, niedermolekulare Urethane und Nebenprodukte der Reaktion, insbesondere aromatische Amine. Hier ist die Abtrennung des Polyetherpolyols besonders einfach.

Die chemische Struktur der entstehenden niedermolekularen Urethane wird durch die in Verfahrensschritt a) verwendeten Reaktanden bestimmt. Die Urethane enthalten einen ersten Kohlenwasserstoffrest, der von der zur Synthese des Polyetherpolyurethans verwendeten Isocyanatkomponente abgeleitet ist. Der zweite Kohlenwasserstoffrest ist von dem in Verfahrensschritt a) eingesetzten niedermolekularen Alkohol abgeleitet. Beide Kohlenwasserstoffreste sind durch eine Urethangruppe verbunden, deren Stickstoffatom an den ersten Kohlenwasserstoffrest gebunden ist.

### Reaktionsbedingungen

Die grundsätzlich geeigneten Reaktionsbedingungen und Katalysatoren für die Umurethanisierung sind in Simon et al. (2018), Waste Management, 76: 147-171 beschrieben. Verfahrensschritt a) wird bei Temperaturen zwischen 140 °C und 300 °C, bevorzugt zwischen 160 °C und 270 °C durchgeführt. Das Gewichtsverhältnis des niedermolekularen Alkohols zum Polyetherurethan liegt zwischen 2 : 1 und 1 : 17. Als Katalysator eignen sich insbesondere Alkalimetall-Hydroxide, Erdalkalimetallhydroxide, Alkalimetallsalze von Carbonsäuren (insbesondere Acetate), Erdalkalimetallsalze von Carbonsäure (insbesondere Acetate), Lewis-Säuren (wie insbesondere Dibutylzinndilaurat), organischen Amine (wie insbesondere Diethanolamin), organometallische Verbindungen (wie insbesondere Titantetrabutanolat) und Zinnverbindungen (wie insbesondere Zinnoctoat). Die Umurethanisierung wird bevorzugt bei Temperaturen im Bereich von 160 °C bis 270 °C in Gegenwart von 0,1 Massen-% bis 5 Massen-prozent Katalysator, bezogen auf die Masse des zugegebenen Polyurethanprodukts.

### Enzymatische Spaltung

Neben der eigentlichen enzymatischen Spaltung des niedermolekularen Urethans kann Verfahrensschritt b) noch weitere Teilschritte enthalten. Diese dienen insbesondere einer verbesserten Effizienz der enzymatischen Spaltung.

Da es sich bei der Spaltung von Urethan um eine Hydrolyse handelt, ist es bevorzugt, das in Verfahrensschritt a) erhaltene Produkt mit Wasser zu versetzen.

In einer bevorzugten Ausführungsform werden aus dem in Verfahrensschritt a) erhaltenen Produkt zunächst die freigesetzten Polyetherpolyole abgetrennt, bevor das zur enzymatischen Spaltung verwendete Enzym zugegeben wird.

Es ist aber ebenfalls möglich, die enzymatische Spaltung in einem Gemisch durchzuführen, das die freigesetzten Polyetherpolyole noch enthält.

In einer weiteren bevorzugten Ausführungsform wird vor der Zugabe des Enzyms der überschüssige, d.h. nicht im niedermolekularen Urethan gebundene Alkohol, abgetrennt. Dies kann besonders vorteilhaft durch Destillation geschehen.

In einer weiteren bevorzugten Ausführungsform wird in Verfahrensschritt b) wenigstens ein Kolöser und/oder wenigstens ein Detergens zugegeben. Hierdurch kann die Löslichkeit des niedermolekularen Urethans in Wasser erhöht und so seine Zugänglichkeit für das Enzym verbessert werden. Bevorzugte Kolöser sind Ethanol, Aceton, Dimethylsulfoxid und Dimethylformamid. Ein bevorzugtes Detergens ist Sorbat.

Das niedermolekulare Urethan kann nach der Umurethanisierung auch durch Salze verunreinigt sein, die bei der enzymatischen Spaltung stören. Deswegen werden in einer weiteren bevorzugten Ausführungsform der vorliegenden Erfindung vor der Zugabe des zur enzymatischen Spaltung verwendeten Enzyms die zusammen mit dem niedermolekularen Urethan vorliegenden Salze ganz oder teilweise abgetrennt.

Da niedermolekulare Alkohole in vielen Fällen keine nachteilige Wirkung auf die Enzymaktivität haben, kann auf diesen Vorbereitungsschritt in vielen Fällen aber auch verzichtet werden. Inwieweit eine Abtrennung des freien niedermolekularen Alkohols vor der enzymatischen Spaltung erforderlich ist, kann der Fachmann durch einfache Vorversuche in Anwesenheit und in Abwesenheit des in Verfahrensschritt a) eingesetzten niedermolekularen Alkohols ermitteln.

Die enzymatische Spaltung in Verfahrensschritt b) kann mit jedem Enzym erfolgen, das Urethanbindungen spalten kann.

Beispielsweise sind hierfür Enzyme mit Aminosäuresequenzen wie in SEQ ID NO: 1 bis 13 definiert geeignet. Besonders gut ist das durch SEQ ID NO: 3 definierte Enzym oder eine Variante davon geeignet. Bei Verwendung der vorgenannten Enzyme ist ein Reaktionspuffer mit 100 mM K₂HPO₄/KH₂PO₄ mit 20 Vol.-% Ethanol bei pH 7 zur enzymatischen Spaltung des niedermolekularen Urethans gut geeignet. Es hat sich aber gezeigt, dass auch ohne Verwendung von Ethanol gute Ergebnisse erzielt werden können.

Eine "Enzym-Variante" wird vorzugsweise durch Hinzufügung, Deletion oder Austausch von bis zu 10 %, stärker bevorzugt bis zu 5 %, der im jeweiligen Polypeptid enthaltenen Aminosäuren erhalten. Grundsätzlich können die vorgenannten Modifikationen an jeder beliebigen Stelle des Polypeptids kontinuierlich oder diskontinuierlich erfolgen. Bevorzugt erfolgen sie aber nur am N-Terminus und/oder am C-Terminus des Polypeptids. Jede erfindungsgemäße durch Hinzufügung, Austausch oder Deletion von Aminosäuren erhaltene Variante ist aber durch Urethanaseaktivität gekennzeichnet. Diese wird bevorzugt durch das Testverfahren gemäß Beispiel 1 nachgewiesen.

Weitere grundsätzlich geeignete Enzyme sind in WO 2006/019095, WO/2013/134801, Shigeno et al. (2006), Applied Microbiology and Biotechnology, 70: 422-429, Gamerith et al. (2016), Polymer degradation and stability, 132: 69-77 und Magnin et al. (2019), Waste management, 85: 141-150 beschrieben.

Als Reaktionsprodukte der enzymatischen Spaltung des niedermolekularen Urethans in Verfahrensschritt b) entsteht der in Verfahrensschritt a) eingesetzte wenigstens eine niedermolekulare Alkohol und ein Amin oder ein Gemisch von Aminen. Die chemische Struktur der entstehenden Amine ist von der Art der zur Synthese des Polyurethans eingesetzten Isocyanatkomponente abhängig. Es werden Amine freigesetzt, die von den in der Isocyanatkomponente eingesetzten Isocyanaten durch Anlagerung von Wasser und nachfolgende Abspaltung von CO₂ abgeleitet werden können.

Das erfindungsgemäße Verfahren liefert somit Verbindungen, deren Strukturen gut definiert sind und die als Ausgangsstoffe für neue Synthesen hochwertiger Produkte geeignet sind.

### Ausführungsbeispiele

### Beispiel 1 (erfindungsgemäß)

### Durchführung:

### Enzymreaktion Modellsubstrate

Die Enzyme mit vermuteter Urethanaseaktivität wurden auf die Hydrolyse von beispielhaften Urethan-Modellverbindungen getestet, um das Substratspektrum zu charakterisieren. Hierbei wurden Carbamate verwendet, die bei der Glykolyse von Polyurethanen gebildet werden können. Alle hierfür verwendeten Modellsubstrate sowie die Screeningsubstrate sind unten dargestellt. Es wurde Reaktionspuffer (100 mM KH₂PO₄/K₂HPO₄, pH 7,0) mit 20% (v/v) Ethanol und 0,2 mg/mL Substrat versetzt. Die Reaktionen wurden in 200-µL-Ansätzen in Glasröhrchen mit Zugabe von 1 -3 mg Enzymlyophilisat durchgeführt. Die Ansätze wurden für ca. 20 h bei Raumtemperatur und anschließend ca. 16 h bei 37 °C schüttelnd inkubiert. Nach der Inkubation wurden die Platten bei Raumtemperatur abgestellt, damit Schwebeteilchen sedimentieren (5 min) und der Überstand über 96-well-Filterplatten mit PVDF-Membran und 0,2 µm Porengröße (Corning, Kaiserslautern) bei 4000 rpm und 20 °C für 5 min in einer Großraumzentrifuge in eine 96-well-Polypropylenplatte zentrifugiert. Die Proben wurden per HPLC mit der Methode "Dabsylamin" vermessen, um das entstehende Amin zu detektieren.

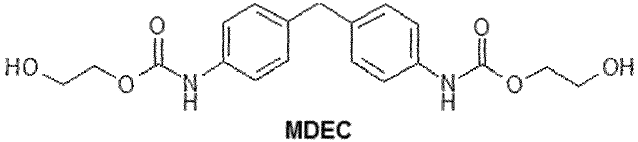

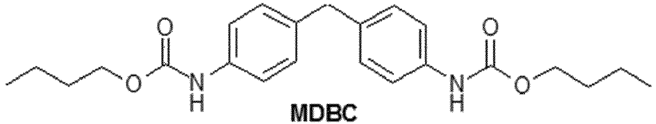

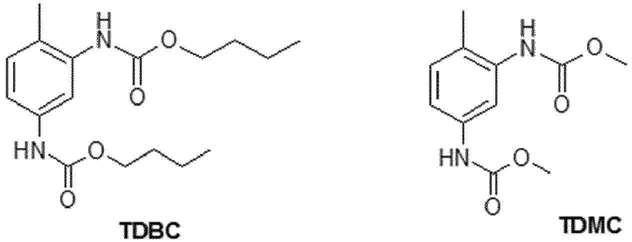

### Enzymreaktionen mit Oligomeren aus PU-Schaum

Weiterhin wurde untersucht, ob die Urethanasen in der Lage sind, lösliche Oligomere, welche bei der Hydrolyse der Esterbindungen eines Polyester-PU-Schaumes entstehen, zu hydrolysieren. Die Glykolyse von von Polyetherpolyurethan mit Diethylenglykol führt zu den gleichen Produkten. Hierfür wurde 1 g des Schaumes mit 20 mL Kaliumphosphatpuffer pH 7,0 und ca. 30 mg CalB-Lyophilisat "Chirazyme L2" (Roche, Basel, Schweiz) in ein 50-mL-Zentrifugenröhrchen gegeben und bei 37 °C und 200 rpm für 5 Tage inkubiert. Anschließend wurde die trübe Lösung bei 25 °C und 4000 rpm in einer Großraumzentrifuge für 10 min zentrifugiert. Der klare Überstand wurde mit 1 M NaOH auf pH 7,0 eingestellt. Nach ca. 6 h bei Raumtemperatur wurde der leicht gefallene pH-Wert erneut auf 7,0 titriert und die Lösung sterilfiltriert.

Hierbei entstanden die unten gezeigten Oligomere. Es ist für jedes Oligomergemisch (OM) jeweils beispielhaft ein Isomer basierend auf 2,4-TDA dargestellt, jedoch befinden sich im Gemisch ebenfalls die Isomere basierend auf 2,6-TDA sowie verschiedene Regioisomere durch abweichende Verknüpfung der Aminogruppen zu Diethylenglykol. Identifizierte Urethanasen wurden auf Hydrolyseaktivität gegenüber diesen Carbamaten getestet.

Die löslichen Oligomere wurden bis zur Verwendung bei 4 °C gelagert. 150 µL dieser Lösung wurden mit 20 µL DMF versetzt. Anschließend wurden jeweils 30 µL der unverdünnten, aufgereinigten Urethanasen zugegeben und die Ansätze bei 30 °C und 1000 rpm auf dem Thermoblock geschüttelt. Als Negativkontrolle diente ein Ansatz mit Enzymlagerpuffer. Nach drei Tagen wurden die Ansätze über Filterplatten mit PVDF-Membran und 0,2 µm Porengröße (Corning, Kaiserslautern) gefiltert und das Filtrat per HPLC mit der Methode "Dabsylamin95" auf entstandenes 2,4- und 2,6-TDA hin untersucht.

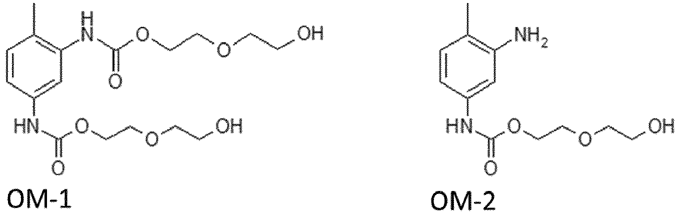

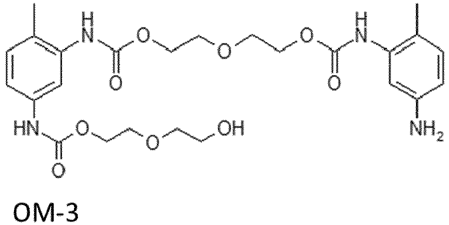

### HPLC-Analyse

Die Hochdruckflüssigchromatographie wurde an einem Gerät der 1100er Serie von Agilent Technologies (Santa Clara, USA) mit *Autosampler* und DAD (*diode array detector*) für UV und den sichtbaren Bereich des Lichtes durchgeführt. Für alle Messungen wurde die Säule "Zorbax XDB-C18" mit einer Partikelgröße von 3,5 µm und den Abmessungen 4,6 x 75 mm (Agilent Technologies, Santa Clara, USA) verwendet. Bei allen Methoden wurden 5 µL Probe injiziert und die Säule auf 40 °C temperiert. Der Fluss lag generell bei 1,5 mL/min. Wegen der Verwendung einer Umkehrphasensäule wurde bei allen Methoden mit steigenden Konzentrationen an organischem Lösemittel eluiert.

Zur Detektion und Quantifizierung von aromatischen Aminen und Urethanen wurden die Methode "Dabsylamin" und "Dabsylamin95" verwendet. Als Laufmittel wurde neben AcN 10 mM Natriumphosphatpuffer pH 7,0 verwendet, dem zum Schutz vor mikrobiellem Wachstum 0,005% (w/v) Natriumazid zugegeben wurde. Die Analyse der Daten erfolgte mit der Software "OpenLAB CDS ChemStationLC" in der Version A.02.09[017] (Agilent Technologies, Santa Clara, USA).

Dabsylamin: Laufmittel: Acetonitril und 10 mM Na₂HPO₄/NaH₂PO₄, pH 7,0

| | |
|---|---|
| t [min] | Acetonitril |
| 0 | 5 |
| 6,5 | 85 |
| 8,0 | 5 |
| 10,0 | 5 |

Dabsylamin95: Laufmittel: Acetonitril mit 5% (v/v) ddH₂O und 10 mM Na₂HPO₄/NaH₂PO₄, pH 7,0

| t [min] | % Acetonitril (+5% (v/v) ddH₂O) |
|---|---|
| 0 | 5 |
| 6,5 | 90 |
| 8,0 | 5 |
| 10,0 | 5 |

### Ergebnisse

### Enzymreaktion Modellsubstrate

Die Modellsubstrate MDEC, MDBC, TDBC und TDMC, die durch chemische Umurethanisierung aus Polyurethanen herstellen lassen, wurden mit aktiven Urethanasen versetzt und für jedes der Modellsubstrate konnte mindestens eine Urethanase gefunden werden, die die hydrolytische Spaltung zum Amin katalysiert (Tabelle 1). Zudem zeigten zwei Urethanasen Aktivität gegenüber den Oligomermischungen OM-1, OM-2 und OM-3, wobei sowohl 2,4-TDA als auch 2,6-TDA freigesetzt wurde.

**Tabelle 1: Zusammenfassung der Untersuchung des Substratspektrums der Urethanasen. Die im Screening mit MDEC deutlich aktiven Enzyme sowie Ure wurden für das Screening von weiteren Urethanverbindungen genutzt. 3: (fast) nur vollständig hydrolysiertes Produkt (Diamin) nachweisbar, 2: Hydrolyse zu Endprodukt und Zwischenprodukt, 1: Hydrolyse zu Zwischenprodukt (Monoamin), 0: (nahezu) keine Hydrolyseprodukte nachweisbar. Mit " - " wurde gekennzeichnet, dass noch deutliche Mengen Substrat vorhanden waren. n.b.: nicht bestimmt. PLE: Schweineleberesterase**

| **Enzym** | **MDEC** | **MDBC** | **TDBC** | **TDMC** | **OM-1** | **OM-2** | **OM-3** |
|---|---|---|---|---|---|---|---|
| Ure (SEQ ID NO. 13) | 0 | 0 | 0 | 1- | 0 | 0 | 0 |
| Lip250 (SEQ ID NO. 9) | 1- | 0 | 0 | 3 | 0 | 0 | 0 |
| Piq liver esterase | 2- | 3- | 3- | 2 | n.b. | n.b. | n.b. |
| *Candida antactica* lipase B | 2- | 0 | 0 | 0 | n.b. | n.b. | n.b. |
| Lip72 (SEQ ID NO. 3) | n.b | n.b | n.b | n.b | 3 | 3- | 3- |
| Lip197 (SEQ ID NO. 7 | n.b | n.b | n.b | n.b | 3- | 3- | 1- |

### Beispiel 2 (erfindungsgemäß): Test unterschiedlicher Reaktionsbedingungen für die Carbamatspaltung mit Aes72

### Einleitung

In diesem Versuch sollten die Carbamate, die nach der chemischen Glykolyse (Umurethanisierung mit Diethylenglykol) von TDI-Weichschaum in der unteren Phase vorkommen, enzymatisch hydrolysiert werden. Durch die Glykolyse des TDI-Weichschaumes wird langkettiges Polyetherpolyol freigesetzt, das sich nach der Reaktion als zweite Phase über dem überschüssigen Glykol und den gebildeten Carbamaten und Aminen absetzt. Die untere Phase des auf diese Weise hergestellten Reaktionsproduktes wurde als Substratlösung verwendet. Durch die enzymatische Hydrolyse der Urethanbindung werden 2,4-TDA und 2,6-TDA sowie Diethylenglykol und CO₂ frei. In Beispiel 1 wurden Cosolventien verwendet. Da zusätzliche Lösemittel in der industriellen Anwendung wieder aufwendig abgetrennt werden müssen, sollte getestet werden, ob die Reaktion auch ohne Cosolventien abläuft. Je höher die Substratkonzentration in der Reaktion ist, desto konzentrierter kann die TDA-Lösung nach der Reaktion sein, was vorteilhaft für die anschließende Aufarbeitung ist. Entsprechend wurde Aes72 (SEQ ID NO. 3) bei Substratkonzentrationen bis 40 % (w/v) getestet. Zudem wurde die Temperatur erhöht, um zu untersuchen, ob dies einen Effekt auf die Reaktionsgeschwindigkeit hat.

### Durchführung

### Chemische Glykolyse

Es wurden 250 g Diethylenglykol vorgelegt und auf 200 °C aufgeheizt. Anschließend wurden 250 g TDI-Weichschaum zudosiert. Die Temperatur wurde nach Auflösung des Schaums noch für drei weitere Stunden konstant gehalten. Als Katalysator wurden 2,5 g Zinn(II)-2-ethylhexanoat verwendet.

### Enzymherstellung

Zur Enzymherstellung wurde *E. coli* BL21(DE3) mit dem Plasmid pET21a-Aes72 transformiert. Sämtliche Kulturen wurden mit 100 mg/L Ampicillin versetzt. Es wurde MagicMedia (Thermofisher) mit einer Einzelkolonie angeimpft und anschließend 24 h bei 30 °C und 130 rpm schüttelnd inkubiert. Die Zellen wurden durch Zentrifugation bei 4000 x g und 4 °C für 10 min abgetrennt. Das Zellpellet wurde in 10 mL 50 mM Kaliumphosphatpuffer pH 7,5 aufgenommen und durch Ultraschall (Amplitude 50 %, Puls 1 s gefolgt von 1 s Pause, 2 min Ultraschallgesamtzeit) aufgeschlossen. Nach der Abtrennung von unlöslichen Bestandteilen durch Zentrifugation (9500 rpm, 4 °C, 20 min) wurde die Rohenzymlösung bei -80 °C eingefroren und anschließend gefriergetrocknet. Das Lyophilisat wurde bei 4 °C gelagert. Zusätzlich wurde eine Leervektorkontrolle als Enzympräparat hergestellt, bei dem der entsprechende Leervektor anstelle von pET21a-Aes72 eingesetzt wurde.

### Enzymreaktion

Enzymlösung wurde durch Lösen von 4,5 % (w/v) Enzymlyophilisat in 50 mM Kaliumphosphatpuffer pH 7,5 hergestellt. Die Ansätze wurden mit einem Gesamtvolumen von 300 µL hergestellt. Es wurde jeweils 60 µL Enzymlösung und 5% (w/v), 10% (w/v), 20% (w/v) und 40% (w/v) der unteren Phase nach der Glykolyse als Substrat eingesetzt. Den restlichen Anteil bildete 50 mM Kaliumphosphatpuffer pH 7,5. Die Inkubation erfolgte bei 40 °C, 50 °C oder 60 °C und 800 rpm im Thermoblock. Reaktionen mit Leervektorkontrollpräparat wurden genauso angesetzt. Nach drei Stunden wurde die Gesamtmenge TDA per HPLC quantifiziert. TDA-Konzentrationen in den Negativkontrollen wurden von den Werten der Enzymreaktionen subtrahiert, um die Menge des durch Aes72 freigesetzten Gesamt-TDAs zu erhalten.

### Abstoppen der Enzymreaktion

Die Proben wurden 1:2 mit 50 mM NaOH in 20 % Essigsäure verdünnt, um die Enzyme zu inaktivieren. Die inaktivierten Proben wurden mindestens 5 min bei Raumtemperatur oder längerfristig bei 4 °C inkubiert und anschließend 1:10 mit 140 mM NaOH verdünnt.

### HPLC-Analytik

Die Analytik des gebildeten 2,4- und 2,6-TDAs erfolgte mittels HPLC. Standards und Proben wurden vor der Analytik zentrifugiert (2 min, 13.300 rpm, Raumtemperatur) und der Überstand wurde durch einen 0,22 µm PES-Filter filtriert. Vom Autosampler wurden jeweils 5 µL Probe injiziert. Als Säule wurde eine ZORBX Eclipse C18 (15 cm) mit entsprechender Vorsäule verwendet. Als Laufmittel A diente Acetonitril, als Laufmittel B 10 mM Natriumphosphatpuffer pH 7,0. Der Gesamtfluss betrug 1 mL/min. Der Laufmittelgradient ist in Tabelle 2 dargestellt.

**Tabelle 1: HPLC-Gradient**

| t [min] | Laufmittel A [%] | Laufmittel B [%] |
|---|---|---|
| 0,00 | 5 | 95 |
| 2,00 | 5 | 95 |
| 10,00 | 95 | 5 |
| 11,00 | 95 | 5 |
| 11,50 | 5 | 95 |
| 16,00 | 5 | 95 |

### Ergebnisse

Die Komponenten, die in der unteren Phase nach der chemischen Glykolyse per NMR quantifiziert wurden, sind in Tabelle3 aufgeführt.

**Tabelle 3: Zusammensetzung der unteren Phase nach der Glykolyse. Die Zusammensetzung wurde durch ¹H-NMR-Messung bestimmt. Die Gewichtsprozent der TDA-Carbamate beziehen sich lediglich auf den TDA-Anteil der Verbindungen, der DEG-Anteil ist als "Carbamatisiertes DEG" aufgeführt.**

| Komponente | Gehalt |
|---|---|
| Polyol | 3,61 Gew.% |
| DEG | 67,2 Gew.% |
| TDA (Diaminitoluol) | 1,92 Gew.% |
| TCA (Toluol-carbamat-amin) | 5,57 Gew.% |
| TDC (Toluoldicarbamat) | 6,01 Gew.% |
| Carbamatisiertes DEG | 15,3 Gew.% |

Die Konzentrationen an freigesetztem TDA unter den verschiedenen Bedingungen in den Enzymreaktionen sind in Tabelle4 gezeigt. Es zeigt sich, dass hohe Umsätze erzielt werden können. Zudem zeigt sich, dass Cosolventien nicht essentiell für die Enzymaktivität sind. Bei allen Temperaturen werden signifikante Mengen TDA freigesetzt, wobei die Reaktionsgeschwindigkeit mit steigender Temperatur bei den beiden niedrigsten Substratkonzentration ansteigt.

**Tabelle 4: Freigesetztes Gesamt-TDA nach 3 h. Die quantifizierte TDA-Menge in den Negativkontrollen wurde von der Menge in den Enzymreaktionen subtrahiert, um die freigesetzte TDA-Menge zu erhalten.**

| T [°C] | Substratkonzentration [% (w/v)] | Freigesetztes TDA [g/L] |
|---|---|---|
| 40 | 5 | 2,93 |
| | 10 | 4,05 |
| | 20 | 2,82 |
| | 40 | 4,80 |
| 50 | 5 | 3,78 |
| | 10 | 4,83 |
| | 20 | 4,51 |
| | 40 | 5,06 |
| 60 | 5 | 4,27 |
| | 10 | 5,07 |
| | 20 | 4,38 |
| | 40 | 2,39 |

### Versuch 3 (Vergleich, nicht erfindungsgemäß): Nachstellung von Beneš et al., 2012 zur Überprüfung der Phasenbildung

### Einleitung

Der nachfolgend beschriebene Versuch soll zeigen, inwieweit die in Beneš, H., Cernä, R., Ďuračková, A., & Lätalovä, P. (2012). Utilization of natural oils for decomposition of polyurethanes. Journal of Polymers and the Environment, 20(1), 175-185 beschriebenen Carbamate als Ausgangsstoffe für das in dieser Anmeldung offenbarte zweistufige Abbauverfahren geeignet sind. Hierfür ist eine möglichst gute Phasentrennung zwischen dem neu hergestellten Carbamat und dem aus dem abgebauten Polyurethan freigesetzten Polyol wesentlich.

Gemäß Beneš et al., 2012 werden Polyetherpolyurethane auf pMDI-Basis mit natürlichen Fetten/Ölen umurethanisiert, wobei das ursprüngliche Polyetherpolyol freigesetzt wird. Da langkettige und hydrophobe Öle wie Rizinusöl im Überschuss eingesetzt werden, ist es unwahrscheinlich, dass nach der Umurethanisierung eine Phasentrennung zwischen dem freigesetzten Polyetherpolyol und den neu gebildeten Carbamaten sowie dem überschüssigen Öl auftritt, wie sie in der vorliegenden Erfindung genutzt wird. Um dies zu überprüfen, wurden die von Beneš et al., 2012 als Produkte beschriebenen Carbamate aus pMDI und Rizinusöl synthesisiert und ein Mischversuch mit dem dort eingesetzten Polyetherpolyol durchgeführt. Dadurch sollte das Produktgemisch nachgestellte werden, wie es nach der dort beschriebenen Glykolyse vorliegt.

### Durchführung

Beispielhaft sollte die Produktmischung aus Run 4BK und 4BK aus Beneš et al., 2012 nachgestellt werden. Zur Synthese wurde eine pMDI Mischung mit 32,25% NCO verwendet. Zu 210 Gewichtsteilen Rizinusöl wurden 7,935 Gewichtsteile pMDI gegeben (10-facher OH-Überschuss) und bei 80 °C gerührt bis ein NCO-Wert von 0,03% gemessen wurde (OH-Zahl = 138,2 mgKOH/g; Viskosität bei 25°C = 1570mPas). Es wurde eine homogene Phase erhalten, in der die MDA-Carbamate vollständig in überschüssigem Rinzinusöl gelöst sind. Zu 26,14 g dieser Carbamatlösung wurden 11,06 g Desmophen 5035 BT (Trifunktionelles Polypropylenetherpolyol mit einer Hydroxylzahl von 35 mg KOH/g, einem Hydroxylgehalt von 1,1 Gew.-% und einem OH-Äquivalentgewicht von ca. 1.600 g, hergestellt von der Covestro Deutschland AG, Leverkusen, Deutschland) gegeben (Verhältnis wie nach Glykolyse in 4BK und 4BK aus D3).

### Ergebnisse

Nach Mischen der der Carbamatlösung und des Polyetherpolyols wurde lediglich eine homogene flüssige Phase beobachtet, die sich auch nach 24 h bei Raumtemperatur nicht entmischte. Dies zeigt, dass das in Beneš et al., 2012 beschriebene Verfahren ungeeignet für eine *split phase* Glykolyse ist. Somit kann das Polyetherpolyol nach der Umurethanisierung nicht als separate Phase abgetrennt werden und es wird auch keine separate Phase der Carbamate mit überschüssigem Glykol erhalten, die ihrerseits zur Wiedergewinnung von aromatischen Aminen genutzt werden kann. Somit ermöglicht das in Beneš et al., 2012 beschriebene Verfahren gerade keine zweistufige Spaltung von Polyurethanen zur Rückgewinnung flexibel einsetzbarer Monomere. Das dortige Produkt ist ein Polyol eigener Art, das nur als Ausgangsstoff für sehr spezifische Reaktionen geeignet ist.

## Patentansprüche

1. Verfahren enthaltend die Schritte
a) Umurethanisierung eines Polyetherpolyurethans mit wenigstens einem niedermolekularen Alkohol mit wenigstens 2 Hydroxylgruppen pro Molekül einem Molekulargewicht von höchstens 500 g/Mol, wobei Polyetherpolyole und niedermolekulare Urethane entstehen; und
b) die enzymatische Spaltung der in Verfahrensschritt a) entstandenen niedermolekularen Urethane, wobei wenigstens ein Amin und der wenigstens eine, in Verfahrensschritt a) eingesetzte niedermolekulare Alkohol freigesetzt werden.

2. Das Verfahren nach Anspruch 1, wobei die Isocyanatkomponente des Polyetherpolyurethans wenigstens ein aromatisches Polyisocyanat enthält.

3. Das Verfahren nach Anspruch 2, wobei das aromatische Polyisocyanat ausgewählt ist aus der Gruppe bestehend aus Toluylendiisocyant (TDI), Methylendiphenylisocyanat (MDI) und Naphthylendiisocyanat.

4. Das Verfahren nach Anspruch 1, wobei die Isocyanatkomponente des Polyetherpolyurethans wenigstens ein aliphatisches Polyisocyanat enthält.

5. Das Verfahren nach Anspruch 4, wobei das aliphatische Polyisocyanat ausgewählt ist aus der Gruppe bestehend aus 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 2,4'- bzw. 4,4'-Diisocyanatodicyclohexylmethan (H12MDI) und 1,10-Diisocyanatodecan.

6. Das Verfahren nach einem der Ansprüche 1 bis 5, wobei wenigstens 50 Gew.-% der zum Aufbau des Polyetherpolyurethans verwendeten Polyolkomponente Bestandteile von Polyetherpolyolen sind.

7. Das Verfahren nach einem der Ansprüche 1 bis 6, wobei der Anteil von Urethan- und Harnstoffbindungen an der Gesamtmenge Urethan-, Harnstoff- und Thiourethanbindungen im Polyetherpolyurethan bei wenigstens 60 Mol-%, bevorzugt wenigstens 80 Mol-% und stärker bevorzugt wenigstens 90 Mol-%.

8. Das Verfahren nach einem der Ansprüche 1 bis 7, wobei der niedermolekulare Alkohol wenigstens 2 Hydroxylgruppen pro Molekül aufweist.

9. Das Verfahren nach einem der Ansprüche 1 bis 8, wobei der niedermolekulare Alkohol einen Schmelzpunkt von höchstens 45 °C hat.

10. Das Verfahren nach einem der Ansprüche 1 bis 9, wobei der niedermolekulare Alkohol ausgewählt ist aus der Gruppe bestehend aus Methanol, Ethanol, Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol, Polyethylenglykol 400 und Mischungen von zwei oder mehr der vorgenannten Alkohole.

11. Das Verfahren nach einem der Ansprüche 1 bis 10, wobei der niedermolekulare Alkohol ausgewählt ist aus der Gruppe bestehend aus Ethylenglykol, Diethylenglykol, Propylenglykol, Dipropylenglykol, Methylglykol, Triethylenglykol und Mischungen von zwei oder mehr der vorgenannten Alkoholen und in einem Mengenverhältnis zum Polyetherpolyurethan eingesetzt wird, so dass am Ende des Verfahrensschritts a) eine vom niedermolekularen Alkohol getrennte Phase vorliegt, welche den freigesetzten Polyether enthält.

12. Das Verfahren nach einem der Ansprüche 1 bis 11, wobei die niedermolekularen Urethane vor Durchführung des Verfahrensschrittes b) von dem wenigstens einen niedermolekularen Alkohol getrennt werden.

13. Das Verfahren nach einem der Ansprüche 1 bis 12, wobei der in Verfahrensschritt b) entstehende niedermolekulare Alkohol erneut in Verfahrensschritt a) eingesetzt wird.

14. Das Verfahren nach einem der Ansprüche 1 bis 13, wobei wenigstens ein Teil des in Verfahrensschritt b) freiwerdenden niedermolekularen Alkohols erneut in Verfahrensschritt a) eingesetzt wird.

## Claims

1. Process comprising the steps of
a) transurethanizing a polyether polyurethane with at least one low-molecular-weight alcohol having at least 2 hydroxyl groups per molecule and a molecular weight of not more than 500 g/mol, forming polyether polyols and low-molecular-weight urethanes; and
b) enzymatically cleaving the low-molecular-weight urethanes formed in process step a), with liberation of at least one amine and the at least one low-molecular-weight alcohol used in process step a).

2. Process according to Claim 1, wherein the isocyanate component of the polyether polyurethane comprises at least one aromatic polyisocyanate.

3. Process according to Claim 2, wherein the aromatic polyisocyanate is selected from the group consisting of tolylene diisocyanate (TDI), methylene diphenyl isocyanate (MDI), and naphthylene diisocyanate.

4. Process according to Claim 1, wherein the isocyanate component of the polyether polyurethane comprises at least one aliphatic polyisocyanate.

5. Process according to Claim 4, wherein the aliphatic polyisocyanate is selected from the group consisting of 1,4-diisocyanatobutane (BDI), 1,5-diisocyanatopentane (PDI), 1,6-diisocyanatohexane (HDI), 2-methyl-1,5-diisocyanatopentane, 1,5-diisocyanato-2,2-dimethylpentane, 2,2,4- or 2,4,4-trimethyl-1,6-diisocyanatohexane, 2,4'- or 4,4'-diisocyanatodicyclohexylmethane (H12MDI), and 1,10-diisocyanatodecane.

6. Process according to any of Claims 1 to 5, wherein at least 50% by weight of the polyol components used to form the polyether polyurethane are constituents of polyether polyols.

7. Process according to any of Claims 1 to 6, wherein the proportion of urethane linkages and urea linkages in the total amount of urethane linkages, urea linkages, and thiourethane linkages in the polyether polyurethane is at least 60 mol%, preferably at least 80 mol%, and more preferably at least 90 mol%.

8. Process according to any of Claims 1 to 7, wherein the low-molecular-weight alcohol has at least 2 hydroxyl groups per molecule.

9. Process according to any of Claims 1 to 8, wherein the low-molecular-weight alcohol has a melting point of not more than 45°C.

10. Process according to any of Claims 1 to 9, wherein the low-molecular-weight alcohol is selected from the group consisting of methanol, ethanol, ethylene glycol, diethylene glycol, propylene glycol, dipropylene glycol, methyl glycol, triethylene glycol, glycerol, 2-methylpropane-1,3-diol, butane-1,4-diol, pentane-1,5-diol, hexane-1,6-diol, polyethylene glycol 400, and mixtures of two or more of the abovementioned alcohols.

11. Process according to any of Claims 1 to 10, wherein the low-molecular-weight alcohol is selected from the group consisting of ethylene glycol, diethylene glycol, propylene glycol, dipropylene glycol, methyl glycol, triethylene glycol, and mixtures of two or more of the abovementioned alcohols and is used in a molar ratio to the polyether polyurethane such that, at the end of process step a), a phase separate from the low-molecular-weight alcohol is present that contains the liberated polyether.

12. Process according to any of Claims 1 to 11, wherein the low-molecular-weight urethanes are separated from the at least one low-molecular-weight alcohol before carrying out process step b).

13. Process according to any of Claims 1 to 12, wherein the low-molecular-weight alcohol formed in process step b) is reused in process step a).

14. Process according to any of Claims 1 to 13, wherein at least part of the low-molecular-weight alcohol liberated in process step b) is reused in process step a) .

## Revendications

1. Procédé contenant les étapes
a) transuréthanisation d'un polyétherpolyuréthane avec au moins un alcool de bas poids moléculaire présentant au moins 2 groupes hydroxyle par molécule d'un poids moléculaire d'au plus 500 g/mole, des polyétherpolyols et des uréthanes de bas poids moléculaire se formant ; et
b) dissociation enzymatique des uréthanes de bas poids moléculaire formés dans l'étape de procédé a), au moins une amine et ledit au moins un alcool de bas poids moléculaire utilisé dans l'étape de procédé a) étant libérés.

2. Procédé selon la revendication 1, le composant isocyanate du polyétherpolyuréthane contenant au moins un polyisocyanate aromatique.

3. Procédé selon la revendication 2, le polyisocyanate aromatique étant choisi dans le groupe constitué par le diisocyanate de toluylène (TDI), l'isocyanate de diphénylméthylène (MDI) et le diisocyanate de naphtylène.

4. Procédé selon la revendication 1, le composant isocyanate du polyétherpolyuréthane contenant au moins un polyisocyanate aliphatique.

5. Procédé selon la revendication 4, le polyisocyanate aliphatique étant choisi dans le groupe constitué par le 1,4-diisocyanatobutane (BDI), le 1,5-diisocyanatopentane (PDI), le 1,6-diisocyanatohexane (HDI), le 2-méthyl-1,5-diisocyanatopentane, le 1,5-diisocyanato-2,2-diméthylpentane, le 2,2,4-triméthyl-1,6-diisocyanatohexane ou le 2,4,4-triméthyl-1,6-diisocyanatohexane, le 2,4'-diisocyanatodicyclohexylméthane ou le 4,4'-diisocyanatodicyclohexylméthane (H12MDI) et le 1,10-diisocyanatodécane.

6. Procédé selon l'une des revendications 1 à 5, au moins 50 % en poids du composant polyol utilisé pour la construction du polyétherpolyuréthane faisant partie de polyétherpolyols.

7. Procédé selon l'une des revendications 1 à 6, la proportion de liaisons uréthane et urée par rapport à la quantité totale de liaisons uréthane, urée et thio-urée dans le polyétherpolyuréthane se situant à au moins 60 % en mole, de préférence au moins 80 % en mole et plus préférablement au moins 90 % en mole.

8. Procédé selon l'une des revendications 1 à 7, l'alcool de bas poids moléculaire présentant au moins 2 groupes hydroxyle par molécule.

9. Procédé selon l'une des revendications 1 à 8, l'alcool de bas poids moléculaire présentant un point de fusion d'au plus 45 °C.

10. Procédé selon l'une des revendications 1 à 9, l'alcool de bas poids moléculaire étant choisi dans le groupe constitué par le méthanol, l'éthanol, l'éthylèneglycol, le diéthylèneglycol, le propylèneglycol, le dipropylèneglycol, le méthylglycol, le triéthylèneglycol, le glycérol, le 2-méthyl-1,3-propanediol, le 1,4-butanediol, le 1,5-pentanediol, le 1,6-hexanediol, le polyéthylèneglycol 400 et les mélanges de deux, ou plus, des alcools susmentionnés.

11. Procédé selon l'une des revendications 1 à 10, l'alcool de bas poids moléculaire étant choisi dans le groupe constitué par l'éthylèneglycol, le diéthylèneglycol, le propylèneglycol, le dipropylèneglycol, le méthylglycol, le triéthylèneglycol et les mélanges de deux, ou plus, des alcools susmentionnés et utilisé en une proportion par rapport au polyétherpolyuréthane telle qu'à la fin de l'étape de procédé a), il existe une phase séparée de l'alcool de bas poids moléculaire, qui contient le polyéther libéré.

12. Procédé selon l'une des revendications 1 à 11, les uréthanes de bas poids moléculaire étant séparés dudit au moins un alcool de bas poids moléculaire avant la réalisation de l'étape de procédé b).

13. Procédé selon l'une des revendications 1 à 12, l'alcool de bas poids moléculaire se formant dans l'étape de procédé b) étant de nouveau utilisé dans l'étape de procédé a) .

14. Procédé selon l'une des revendications 1 à 13, au moins une partie de l'alcool de bas poids moléculaire se libérant dans l'étape de procédé b) étant de nouveau utilisée dans l'étape de procédé a).
